(19) Europäisches Patentamt
European Patent Office
Office européen des brevets

(11) EP 4 098 263 A1

(12) **EUROPEAN PATENT APPLICATION**
published in accordance with Art. 153(4) EPC

(43) Date of publication:
**07.12.2022 Bulletin 2022/49**

(21) Application number: **21747642.3**

(22) Date of filing: **29.01.2021**

(51) International Patent Classification (IPC):
***A61K 31/519** (2006.01)* ***A61P 19/08** (2006.01)*
***C07K 14/715** (2006.01)*

(52) Cooperative Patent Classification (CPC):
**A61K 31/519; A61P 19/08; C07K 14/715**

(86) International application number:
**PCT/JP2021/003133**

(87) International publication number:
**WO 2021/153703 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2020 JP 2020014260**

(71) Applicants:
- **Kyoto University**
  **Kyoto-shi, Kyoto 606-8501 (JP)**
- **Taiho Pharmaceutical Co., Ltd.**
  **Chiyoda-ku, Tokyo 101-8444 (JP)**

(72) Inventors:
- **TSUMAKI, Noriyuki**
  **Kyoto-shi, Kyoto 606-8501 (JP)**
- **IIMORI, Yuki**
  **Kyoto-shi, Kyoto 606-8501 (JP)**
- **MIURA, Akihiro**
  **Tsukuba-shi, Ibaraki 300-2611 (JP)**
- **HIRAI, Hiroshi**
  **Tsukuba-shi, Ibaraki 300-2611 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **TREATMENT FOR CHONDRODYSTROPHIA**

(57) A pharmaceutical composition for treating cartilage dysplasia, comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof, and a treatment method using the pharmaceutical composition.

DRAWINGS

Fig. 1

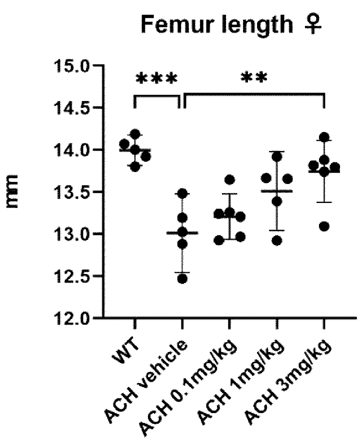


EP 4 098 263 A1

## Description

Technical Field

Cross-Reference to Related Application

This application claims priority based on Japanese Patent Application No. 2020-014260 filed on January 31, 2020, the entire contents of which are incorporated herein by reference.

The present invention relates to treatment of cartilage dysplasia (achondroplasia, cartilage hypoplasia, thanatophoric dysplasia) using an FGFR inhibitor.

Background Art

Fibroblast growth factors (FGFs) are expressed in various tissues, and are one of the growth factors that regulate cell proliferation and differentiation. The physiological activity of the FGFs is mediated by fibroblast growth factor receptors (FGFRs), which are specific cell surface receptors. FGFRs belong to a receptor protein tyrosine kinase family, and comprise an extracellular ligand-binding domain, a single transmembrane domain, and an intracellular tyrosine kinase domain. Four types of FGFRs (FGFR1, FGFR2, FGFR3, and FGFR4) have been heretofore identified. FGFRs bind to FGFs to form dimers, and are activated by phosphorylation. Activation of the receptors induces mobilization and activation of specific downstream signal transduction molecules, thereby developing physiological functions.

Some reports have been made about the relationship between aberrant FGF/FGFR signaling and diseases related to abnormal chondrocyte differentiation in humans. Aberrant activation of FGF/FGFR signaling in diseases related to abnormal chondrocyte differentiation in humans is considered to be attributable to gene mutation of FGFRs (NPL 1 and NPL 2).

In cartilage dysplasia, point mutations such as G380R, N540K, and K650E in FGFR3 have been reported, and it is suggested that such gene mutations may be a cause of cartilage dysplasia (NPL 3, 4, and 5). Additionally, NPL 6 reported that the FGFR inhibitor NVP-BGJ398 has a treatment effect on an $FGFR3^{Y367C/+}$ mouse model of achondroplasia, wherein the $FGFR3^{Y367C/+}$ is an activating mutation outside the kinase domain. Further, there is a report indicating that the NVP-BGJ398 has a kinase inhibitory effect on the mutant $FGFR3^{G380R/+}$ at 50 nM. In contrast, there is no report about a treatment effect on the $FGFR3^{G380R/+}$ mouse model of achondroplasia.

PTL 1 reported about disubstituted benzene alkynyl compounds having an FGFR inhibitory effect. PTL 2 and 3 respectively reported that these compounds are effective against cancer with a specific FGFR2 mutation, and that an intermittent administration may be effective on the administration schedule.

Citation List

Patent Literature

PTL 1: WO2013/108809
PTL 2: WO2015/008844
PTL 3: WO2015/008839

Non-patent Literature

NPL 1: Dev. Dyn. 2017 Apr; 246 (4): 291-309
NPL 2: Am. J. Hum. Genet. 2000 Dec; 67 (6): 1411-21
NPL 3: Nature. 1994 Sep 15; 371 (6494): 252-4
NPL 4: Nat. Genet. 1995 Jul; 10 (3): 357-9
NPL 5: Am. J. Med. Genet. 1996 May 3; 63 (1): 148-54
NPL 6: J. Clin. Invest. 2016 126 (5): 1871-1884

Summary of Invention

Technical Problem

The present invention aims to provide a novel medicament for treating cartilage dysplasia, and a treatment method using the pharmaceutical composition.

Solution to Problem

As a result of extensive research to attain the above object, the present inventors found that 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one (Futibatinib) or a pharmaceutically acceptable salt thereof inhibits phosphorylation of mutant FGFR3, and has an excellent bone extension effect against cartilage dysplasia.

Specifically, the present invention includes the following Items [1] to [11].

[1] A pharmaceutical composition for treating cartilage dysplasia, comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof.

[2] The pharmaceutical composition according to Item 1, wherein the cartilage dysplasia is achondroplasia, cartilage hypoplasia, or thanatophoric dysplasia.

[3] The pharmaceutical composition according to Item 1 or 2, wherein the cartilage dysplasia is achondroplasia.

[4] The pharmaceutical composition according to any one of Items 1 to 3, wherein the cartilage dysplasia is cartilage dysplasia having an FGFR3 mutation.

[5] The pharmaceutical composition according to Item [4], wherein the FGFR3 mutation is a mutation of the $248^{th}$ arginine, $380^{th}$ glycine, $540^{th}$ asparagine, or $650^{th}$ lysine in the FGFR3.

[6] The pharmaceutical composition according to Item [4], wherein the cartilage dysplasia having an FGFR3 mutation is achondroplasia having an FGFR3 mutation.

[7] The pharmaceutical composition according to Item [6], wherein the achondroplasia is achondroplasia having an FGFR3 mutation in which the $380^{th}$ glycine in the FGFR3 is mutated to arginine.

[8] The pharmaceutical composition according to Item [4], wherein the cartilage dysplasia having an FGFR3 mutation is cartilage hypoplasia having an FGFR3 mutation.

[9] The pharmaceutical composition according to Item 8, wherein the cartilage hypoplasia is cartilage hypoplasia having an FGFR3 mutation in which the $540^{th}$ asparagine in the FGFR3 is mutated to lysine.

[10] The pharmaceutical composition according to Item 4, wherein the cartilage dysplasia having an FGFR3 mutation is thanatophoric dysplasia having an FGFR3 mutation.

[11] The pharmaceutical composition according to Item [10], wherein the thanatophoric dysplasia is thanatophoric dysplasia having an FGFR3 mutation in which the $248^{th}$ arginine in the FGFR3 is mutated to cysteine, or an FGFR3 mutation in which the $650^{th}$ lysine is mutated to glutamic acid.

The present invention also relates to the following embodiments.

- An agent for cartilage dysplasia treatment comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof.
- 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof for use in the treatment of cartilage dysplasia.
- Use of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof for the treatment of cartilage dysplasia.
- Use of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof in the manufacture of a medicament for the treatment of cartilage dysplasia.
- A method of treating cartilage dysplasia, comprising the step of administering an effective amount of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof to a cartilage dysplasia patient.
- A commercial package comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof as an active ingredient, together with instructions for using it for the treatment of cartilage dysplasia in a subject.

Advantageous Effects of Invention

The present invention ensures a treatment having an excellent bone extension effect on cartilage dysplasia.

Brief Description of Drawings

Fig. 1 shows the measurement results of femur length. The vertical axis indicates the bone length (mm). The horizontal axis is as follows. WT: Wild-type mouse; ACH vehicle: ACH mouse, vehicle administration group; ACH 0.1 mg/kg: ACH mouse, 0.1 mg/kg administration group; ACH 1 mg/kg: ACH mouse, 1 mg/kg administration group; ACH 3 mg/kg: ACH mouse, 3 mg/kg administration group.

Fig. 2 shows the measurement results of tibia length. The vertical axis indicates the bone length (mm). The horizontal axis is as follows. WT: Wild-type mouse; ACH vehicle: ACH mouse, vehicle administration group; ACH 0.1 mg/kg: ACH mouse, 0.1 mg/kg administration group; ACH 1 mg/kg: ACH mouse, 1 mg/kg administration group; ACH 3 mg/kg: ACH mouse, 3 mg/kg administration group.

Fig. 3 shows the measurement results of ulnar length. The vertical axis indicates the bone length (mm). The horizontal axis is as follows. WT: Wild-type mouse; ACH vehicle: ACH mouse, vehicle administration group; ACH 0.1 mg/kg: ACH mouse, 0.1 mg/kg administration group; ACH 1 mg/kg: ACH mouse, 1 mg/kg administration group; ACH 3 mg/kg: ACH mouse, 3 mg/kg administration group.

Fig. 4 shows the measurement results of the thickness of femur growth plate cartilage. The vertical axis indicates the thickness of growth plate cartilage (pm). The horizontal axis is as follows. WT: Wild-type mouse, ACH vehicle: ACH mouse, vehicle administration group; ACH 0.1 mg/kg: ACH mouse, 0.1 mg/kg administration group; ACH 1 mg/kg: ACH mouse, 1 mg/kg administration group; ACH 3 mg/kg: ACH mouse, 3 mg/kg administration group.

Fig. 5 shows the measurement results of the thickness of tibia growth plate cartilage. The vertical axis indicates the thickness of growth plate cartilage (pm). The horizontal axis is as follows. WT: Wild-type mouse; ACH vehicle: ACH mouse, vehicle administration group; ACH 0.1 mg/kg: ACH mouse, 0.1 mg/kg administration group; ACH 1 mg/kg: ACH mouse, 1 mg/kg administration group; ACH 3 mg/kg: ACH mouse, 3 mg/kg administration group.

Fig. 6 shows the measurement results of femur length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/kg administration group; ACH 10 mg/kg: ACH mouse, 10 mg/kg administration group.

Fig. 7 shows the measurement results of tibia length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/kg administration group; ACH 10 mg/kg: ACH mouse, 10 mg/kg administration group.

Fig. 8 shows the measurement results of ulnar length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/kg administration group; ACH 10 mg/kg: ACH mouse, 10 mg/kg administration group.

Fig. 9 shows the measurement results of femur length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/kg administration group; ACH 6 mg/kg: ACH mouse, 6 mg/kg administration group.

Fig. 10 shows the measurement results of tibia length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/kg administration group; ACH 6 mg/kg: ACH mouse, 6 mg/kg administration group.

Fig. 11 shows the measurement results of ulnar length. The vertical axis indicates the bone length (mm). The horizontal axis is as follows. WT: Wild-type mouse; ACH vehicle: ACH mouse, vehicle administration group; ACH 1 mg/kg: ACH mouse, 1 mg/kg administration group; ACH 3 mg/kg: ACH mouse, 3 mg/g administration group; ACH 6 mg/kg: ACH mouse, 6 mg/kg administration group.

Fig. 12 shows the drug efficacy evaluation results (Safranin staining diagram) using iPS cells derived from patients with thanatophoric dysplasia type I (TD1) and cartilage intangibility (ACH), and glucosaminoglycan as an index. Vehicle: vehicle (=0.1% DMSO) administration group; Compound 1 1 nM: 1 nM, Compound 1 administration group.

Fig. 13 shows the evaluation results of drug efficacy using iPS cells derived from patients with thanatophoric dysplasia type I (TD1) and cartilage intangibility (ACH), and the expression of mRNAs of COL2A and ACAN as an index. Measurement was performed using a Step One Plus Real-Time PCR System (Applied Biosystems). The vertical

axis shows the ΔCt value using a GAPDH gene as an internal standard. The horizontal axis is as follows. Vehicle: vehicle (=0.1% DMSO) administration group; Compound 1: 1 nM, Compound 1 administration group.

Description of Embodiments

The present invention relates to an agent for cartilage dysplasia treatment comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof; a pharmaceutical composition for treating cartilage dysplasia, comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof as an active ingredient; and a treatment method using the pharmaceutical composition.

1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one (hereinbelow referred to as "Compound 1" in this specification) is a disubstituted benzene alkynyl compound having the following structure. For example, the compound can be synthesized according to the production method described in WO2013/108809, although the method is not particularly limited.

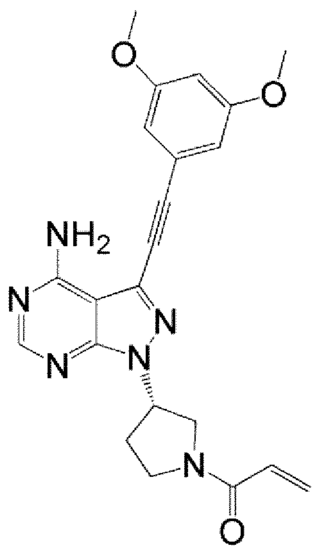

In the present invention, Compound 1 can be used as is, or in the form of a pharmaceutically acceptable salt. The pharmaceutically acceptable salt of Compound 1 is not particularly limited, and examples include addition salts with inorganic acids such as hydrochloric acid and sulfuric acid, or with organic acids such as acetic acid, citric acid, tartaric acid, and maleic acid; salts with alkali metals, such as potassium and sodium; salts with alkaline earth metals, such as calcium and magnesium; salts with organic bases, such as ammonium salts, ethylamine salts, and arginine salts; and the like.

In the present invention, "FGFR3" includes FGFR3 of humans or non-human mammals; FGFR3 of humans is preferred. The NCBI Gene ID number of human FGFR3 is 2261. Further, an FGFR3 protein comprises its isoforms having splicing variant thereof. Examples of a human-derived isoform include a polypeptide consisting of the amino acid sequence (SEQ ID No. 1) encoded by the NCBI Reference Sequence: NP-000133.

In the present invention, the "FGFR3 mutation" is not particularly limited as long as it is an FGFR3 protein having an amino acid mutation that causes cartilage dysplasia, or an FGFR3 gene encoding the amino acid. In the case of an FGFR3 protein having an amino acid sequence in which at least one amino acid selected from the group consisting of the 248$^{th}$ arginine, 380$^{th}$ glycine, 540$^{th}$ asparagine, and 650$^{th}$ lysine in the amino acid sequence represented by SEQ ID No. 1 is mutated, or in the case of an FGFR3 protein having a wild-type amino acid sequence different from SEQ ID No. 1, an FGFR3 protein having an amino acid sequence in which at least one amino acid at a position corresponding to the above position in SEQ ID No. 1 is mutated, or an FGFR3 gene encoding the amino acid sequence is preferred.

In the case of an FGFR3 protein having an amino acid sequence in which at least one amino acid selected from the group consisting of the 248$^{th}$ arginine, 380$^{th}$ glycine, and 650$^{th}$ lysine in the amino acid sequence represented by SEQ ID No. 1 is mutated, or in the case of an FGFR3 protein having a wild-type amino acid sequence different from SEQ ID No. 1, an FGFR3 protein having an amino acid sequence in which at least one amino acid at a position corresponding to the above position in SEQ ID No. 1 is mutated, or an FGFR3 gene encoding the amino acid sequence is more preferred.

In the case of an FGFR3 protein having an amino acid sequence in which at least one amino acid selected from the group consisting of the 248$^{th}$ arginine and 380$^{th}$ glycine in the amino acid sequence represented by SEQ ID No. 1 is mutated, or in the case of an FGFR3 protein having a wild-type amino acid sequence different from SEQ ID No. 1, an FGFR3 protein having an amino acid sequence in which at least one amino acid at a position corresponding to the above position in SEQ ID No. 1 is mutated, or an FGFR3 gene encoding the amino acid sequence is still more preferred.

In the case of an FGFR3 protein having an amino acid sequence in which the 380$^{th}$ glycine in the amino acid sequence represented by SEQ ID No. 1 is mutated, or in the case of an FGFR3 protein having a wild-type amino acid sequence different from SEQ ID No. 1, an FGFR3 protein having an amino acid sequence in which glycine at the 380$^{th}$ position in SEQ ID No. 1 is mutated, or an FGFR3 gene encoding the amino acid sequence is even more preferred.

In the present specification, regarding a certain FGFR3 protein, the X$^{th}$ amino acid represented by SEQ ID No. 1 means, unless otherwise specified, the X$^{th}$ amino acid in the amino acid sequence represented by SEQ ID No. 1, and an amino acid at a position corresponding to the X$^{th}$ position in SEQ ID No. 1 in the case of an FGFR3 protein having a wild-type amino acid sequence different from SEQ ID No. 1. To which position of the amino acid represented by SEQ ID No. 1 a certain amino acid of FGFR3 protein having a wild-type amino acid sequence different from that of SEQ ID No. 1 corresponds can be confirmed, for example, by Multiple Alignment of BLAST.

As FGFR3 in which the 380$^{th}$ glycine or glycine at a position corresponding to the 380$^{th}$ position in SEQ ID No. 1 is mutated, FGFR3 in which the 380$^{th}$ glycine or glycine at a corresponding position is mutated to arginine is preferred. In this specification, FGFR3 in which the 380$^{th}$ glycine or glycine at a position corresponding to the 380$^{th}$ position in SEQ ID No. 1 is mutated is sometimes referred to as G380R. The same applies to R248C, N540K, and K650E described below. As FGFR3 in which the 248$^{th}$ arginine or arginine at a position corresponding to the 248$^{th}$ position in SEQ ID No. 1 is mutated, R248C in which the 248$^{th}$ arginine or arginine at a corresponding position is mutated to cysteine is preferred. As FGFR3 in which the 540$^{th}$ asparagine or asparagine at a position corresponding to the 540$^{th}$ position in SEQ ID No. 1 is mutated, N540K in which the 540$^{th}$ asparagine or asparagine at a corresponding position is mutated to lysine is preferred. As FGFR3 in which the 650$^{th}$ lysine or lysine at a position corresponding to the 650$^{th}$ position in SEQ ID No. 1 is mutated, K650E in which the 650$^{th}$ lysine or lysine at a corresponding position is mutated to glutamic acid is preferred.

The FGFR3 mutation is preferably an FGFR3 protein having an amino acid sequence containing at least one amino acid mutation selected from the group consisting of R248C, G380R, N540K, and K650E, or an FGFR3 gene encoding the amino acid sequence; more preferably an FGFR3 protein having an amino acid sequence containing at least one amino acid mutation selected from the group consisting of R248C, G380R, and K650E, or an FGFR3 gene encoding the amino acid sequence; still more preferably an FGFR3 protein having an amino acid sequence containing at least one amino acid mutation selected from the group consisting of R248C and G380R, or an FGFR3 gene encoding the amino acid sequence; and even more preferably an FGFR3 protein having a G380R mutation or an FGFR3 gene encoding the amino acid sequence.

As mentioned above, in a mutation in certain FGFR3 isoforms, even if the position of the mutation is different from the position of the amino acid represented by SEQ ID No. 1 due to the deletion, insertion, or the like of the amino acid, the mutation is considered to be the same as a position corresponding to the position of the amino acid represented by SEQ ID No. 1. Accordingly, for example, the 380$^{th}$ glycine in FGFR3 represented by SEQ ID No. 1 corresponds to the 382$^{nd}$ glycine in FGFR3 having the amino acid sequence (SEQ ID No. 2) represented by NCBI Reference Sequence: NP_001156685. Accordingly, unless otherwise specified, in the present invention, "G380R" means that the 380$^{th}$ glycine in FGFR3 represented by SEQ ID No. 1 is mutated to arginine, as well as that the 382$^{nd}$ glycine in FGFR3 having an amino acid sequence represented by the NCBI Reference Sequence: NP_001156685 is mutated to arginine. To which position of the amino acid represented by SEQ ID No. 1 a certain amino acid of a certain FGFR3 isoform corresponds can be confirmed, for example, by Multiple Alignment of BLAST.

In the present invention, "cartilage dysplasia" means diseases caused by reduced function of growth cartilage, such as achondroplasia, cartilage hypoplasia, and thanatophoric dysplasia; preferably achondroplasia, cartilage hypoplasia, and thanatophoric dysplasia; and more preferably achondroplasia.

In the present invention, "cartilage dysplasia having an FGFR3 mutation" means a disease caused by reduced function of growth cartilage due to a mutation of the 380$^{th}$ glycine in SEQ ID No. 1 or glycine at a position corresponding to the 380$^{th}$ position in SEQ ID No. 1; the 540$^{th}$ asparagine or asparagine at a position corresponding to the 540$^{th}$ position in SEQ ID No. 1; the 248$^{th}$ arginine or arginine at a position corresponding to the 248$^{th}$ position in SEQ ID No. 1; or the 650$^{th}$ lysine or lysine at a position corresponding to the 650$^{th}$ position in SEQ ID No. 1.

Preferable examples include achondroplasia having an FGFR3 mutation in which the 380$^{th}$ glycine or glycine at a position corresponding to the 380$^{th}$ position in SEQ ID No. 1 is mutated to arginine; cartilage hypoplasia having an FGFR3 mutation in which the 540$^{th}$ asparagine or asparagine at a position corresponding to the 540$^{th}$ position in SEQ ID No. 1 is mutated to lysine; and thanatophoric dysplasia having an FGFR3 mutation in which the 248$^{th}$ arginine or arginine at a position corresponding to the 248$^{th}$ position in SEQ ID No. 1 is mutated to cysteine, or an FGFR3 mutation in which the 650$^{th}$ lysine or lysine at a position corresponding to the 650$^{th}$ position in SEQ ID No. 1 is mutated to glutamic acid.

More preferable examples include achondroplasia having an FGFR3 mutation in which the $380^{th}$ glycine or glycine at a position corresponding to the $380^{th}$ position in SEQ ID No. 1 is mutated to arginine; and thanatophoric dysplasia having an FGFR3 mutation in which the $248^{th}$ arginine or arginine at a position corresponding to the $248^{th}$ position in SEQ ID No. 1 is mutated to cysteine, or an FGFR3 mutation in which the $650^{th}$ lysine or lysine at a position corresponding to the $650^{th}$ position in SEQ ID No. 1 is mutated to glutamic acid.

Even more preferable examples include achondroplasia having an FGFR3 mutation in which the $380^{th}$ glycine or glycine at a position corresponding to the $380^{th}$ position in SEQ ID No. 1 is mutated to arginine; and thanatophoric dysplasia having an FGFR3 mutation in which the $248^{th}$ arginine or arginine at a position corresponding to the $248^{th}$ position in SEQ ID No. 1 is mutated to cysteine.

Still more preferable examples include achondroplasia having an FGFR3 mutation in which the $380^{th}$ glycine or glycine at a position corresponding to the $380^{th}$ position in SEQ ID No. 1 is mutated to arginine.

In this specification, cartilage dysplasia having an FGFR3 mutation may sometimes simply be referred to as FGFR3 mutation cartilage dysplasia.

In the present invention, the FGFR3 mutation can be detected by a method that is well-known to those skilled in the art. Examples of the method of detecting a mutation of FGFR3 gene include conventionally known methods, such as Southern blotting, PCR, DNA microarray, and sequencing analysis. Examples of the method of detecting a mutation of FGFR3 protein include conventionally known methods, such as methods using an antibody that specifically binds to a FGFR3 mutation (ELISA, Western blotting, immunostaining, etc.), and mass spectral analysis. As the antibody that specifically binds to the FGFR3 mutation, a commercially available product can be used. Alternatively, the antibody can be produced by a conventionally known method.

In the present invention, the term "sample" includes not only a biological sample (e.g., cells, tissues, organs, body fluids (blood, lymph fluid, and the like), digestive fluid, urine), but also a nucleic acid extract (e.g., genomic DNA extracts, mRNA extracts, cDNA preparation and cRNA preparation prepared from mRNA extracts, and the like) and a protein extract obtained from these biological samples. Further, the sample may be subjected to a formalin fixation treatment, an alcohol fixation treatment, a freezing treatment, or a paraffin embedding treatment. As the biological sample, a sample obtained from a living body can be used. The method for obtaining a biological sample can be suitably selected, depending on the type of biological sample.

In the present invention, Compound 1 or a pharmaceutically acceptable salt thereof can be used, as is, as an agent for cartilage dysplasia treatment; or a combination of Compound 1 or a pharmaceutically acceptable salt thereof with a pharmaceutical carrier can be used as a pharmaceutical composition. Accordingly, in one embodiment, the present invention provides a pharmaceutical composition containing Compound 1 or a pharmaceutically acceptable salt thereof.

When Compound 1 or a pharmaceutically acceptable salt thereof is incorporated in a formulation as an active ingredient, a pharmaceutical carrier can be optionally added, thereby forming a suitable dosage form according to prevention and treatment purposes. Examples of the dosage form include oral preparations, injections, suppositories, ointments, patches, and the like. Of these, oral preparations are preferable. Examples of oral preparations include tablets, capsules, granules, powders, syrups, and the like, without any limitation. Such dosage forms can be formed by methods conventionally known to persons skilled in the art. A suitable carrier, such as an excipient, diluent, bulking agent, or disintegrant, can be optionally added to the formulation or pharmaceutical composition according to the dosage form.

The amount of Compound 1 or a pharmaceutically acceptable salt thereof to be incorporated in each of such dosage unit forms depends on the condition of the patient to whom Compound 1 or its salt is administered, the dosage form, etc. In general, in the case of an oral preparation, an injection, and a suppository, the amount is preferably 0.05 to 1000 mg, 0.01 to 500 mg, and 1 to 1000 mg, respectively, per dosage unit form.

The dose of Compound 1 or a pharmaceutically acceptable salt thereof per day depends on the condition, body weight, age, gender, etc. of the patient, and cannot be generalized. For example, the dose of Compound 1 or a pharmaceutically acceptable salt thereof for an adult (body weight: 60 kg) per day is typically about 1 to 1000 mg, preferably about 10 to 500 mg, and more preferably about 10 to 300 mg.

When Compound 1 or a pharmaceutically acceptable salt thereof is administered every day, the dose of Compound 1 or a pharmaceutically acceptable salt thereof is, for example, about 1 to 200 mg, preferably 2 to 100 mg, more preferably 4 to 50 mg, and even more preferably 10 to 40 mg, per day.

When Compound 1 or a pharmaceutically acceptable salt thereof is administered intermittently, the dose of Compound 1 or a pharmaceutically acceptable salt thereof is, for example, about 2 to 1000 mg, preferably 10 to 500 mg, more preferably 20 to 200 mg, and even more preferably 50 to 160 mg, per day.

Regarding the administration schedule, Compound 1 or its pharmaceutically acceptable salt can be administered every day or intermittently.

In this specification, "administered every day" may be an administration schedule based on a cycle in which dosing is performed for 21 days every day (one cycle), and a period of drug holidays may be provided as each cycle ends.

In this specification, "administered intermittently" is not particularly limited as long as the conditions of at least twice a week and a dosing interval of at least one day between dosing (the number of days between a certain dosing

date and the next dosing date) are satisfied.

Examples include an administration schedule based on a 1-week cycle, in which Compound 1 or a pharmaceutically acceptable salt thereof is administered at least twice every one to three days per cycle (with a dosing interval between a certain dosing date and the next dosing date of 1 to 3 days), and this cycle is performed once or repeated twice or more; an administration schedule based on a 14-day cycle, in which Compound 1 or a pharmaceutically acceptable salt thereof is administered 4 to 7 times with a dosing interval between a certain dosing date and the next dosing date of 1 to 3 days, and this cycle is performed once or repeated twice or more; an administration schedule based on a 14-day cycle, in which, among 14 days contained in one cycle, Compound 1 or a pharmaceutically acceptable salt thereof is administered on Day 1, Day 4, Day 8, and Day 11; an administration schedule based on a 14-day cycle, in which among 14 days contained in one cycle, Compound 1 or a pharmaceutically acceptable salt thereof is administered on Day 1, Day 3, Day 5, Day 7, Day 9, Day 11, and Day 13; and an administration schedule based on a 14-day cycle, in which, among 14 days contained in one cycle, Compound 1 or a pharmaceutically acceptable salt thereof is administered on Day 1, Day 3, Day 5, Day 8, Day 10, and Day 12. In the present invention, "with a dosing interval between a certain dosing date and the next dosing date of X days" means that if the administration is given on Day n, the date of next administration shall be Day n+(1+X). For example, an interval of 1 day between a certain dosing date and the next dosing date means that when the dosing is performed on Day 1, the next dosing is performed on Day 3.

The present invention also provides a method of treating cartilage dysplasia comprising the step of administering an effective amount of Compound 1 or a pharmaceutically acceptable salt thereof to a patient of cartilage dysplasia. Compound 1 or a pharmaceutically acceptable salt thereof, the administration method thereof, etc. are as described above. Examples of the patient include humans, non-human mammals, and the like. Examples of non-human mammals include monkeys, dogs, cats, rabbits, mice, rats, guinea pigs, and the like. Further, as described above, examples of cartilage dysplasia include FGFR3 mutation cartilage dysplasia etc.

Accordingly, in one embodiment, the present invention also provides a method of treating FGFR3 mutation cartilage dysplasia, comprising the following steps (1) and (2):

(1) detecting a mutation of FGFR3 protein or FGFR3 gene from a sample derived from a patient; and
(2) administering an effective amount of Compound 1 or a pharmaceutically acceptable salt thereof to the patient to whom the mutation of FGFR3 protein or FGFR3 gene has been detected in step (1) above.

In one embodiment, the present invention also provides the following method:

a method of treating FGFR3 mutation cartilage dysplasia,
comprising the step of administering an effective amount of Compound 1 or a pharmaceutically acceptable salt thereof to a patient having a mutation of FGFR3 protein or FGFR3 gene, wherein the mutation of FGFR3 protein or FGFR3 gene is detected from a sample derived from the patient.

In the above treatment method, it is assumed that a chemotherapy in which an effective amount of Compound 1 or a pharmaceutically acceptable salt thereof is administered has a sufficient treatment effect on a patient from whom the mutation of FGFR3 protein or FGFR3 gene is detected. Here, "the treatment effect" can be evaluated by the bone extension effect. The treatment effect can also be estimated by the degree of function inhibitory activity of FGFR3 (e.g., inhibitory activity using FGFR3 phosphorylation as an index).

## Examples

The present invention is detailed below with reference to Examples; however, the present invention is not limited thereto. The present invention is fully described below by way of Examples; however, it can be understood that various changes and modifications by a skilled artisan are possible. Therefore, such changes and modifications are included in the present invention as long as they do not depart from the scope of the invention.

### Example 1: Evaluation of inhibitory activity of Compound 1 against FGFR3 mutant *in vitro*

#### 1-1 Construction of FGFR3 mutant expression vector

As an FGFR3 vector, FGFR3 (NM_000142) Human Tagged ORF Clone (FGFR3 wild-type (WT) expression vector) purchased from ORIGENE was used. Vectors for expressing respective mutants (G380R, N540K, and K650E) were constructed using the above vector as a template.

1-2 Measurement of inhibitory activity of FGFR3 using FGFR3 phosphorylation as an index

Human embryonic kidney cells (HEK293T) were cultured in a DMEM containing 10% fetal bovine serum. After the cells were collected by a normal method, they were suspended in a DMEM containing 10% fetal bovine serum. According to the lipotransfection method using a Lipofectamine 3000 reagent (Thermo Fisher Scientific), the FGFR3 wild-type vector or FGFR3 mutant expression vectors mentioned above were individually transfected into the cells. The cells were then seeded at $1.5 \times 10^4$ cells/100 μL per well in a 96-well plate.

As a drug solution, a vehicle (DMSO) group and a diluent series (diluent series having 9 concentrations, including 3000 nM as the maximum final concentration, 1000, 300, 100, 30, 10, 3, 1, 0.3 nM) of Compound 1 were prepared. The seeded cells were incubated at 37°C, 5% $CO_2$ for 24 hours, and then 11 μL of medium containing a drug solution was added thereto, followed by incubation for another one hour.

Functional inhibition against the autophosphorylation ability of FGFR3 was measured and evaluated using a Human Phospho-FGFR3 DuoSet IC ELISA (R&D Systems). Specifically, a protease inhibitor (Roche) and a phosphatase inhibitor (Roche) were added to a cell lysis buffer attached to the kit, and the cells were lysed using them. An experiment was conducted according to the protocol of the kit. Colorimetric quantification was performed on each well using a plate reader (Spectra MAX384, Molecular Devices). The relative FGFR3 phosphorylation percentage in a drug solution-added well was calculated according to the following formula using the control group as 100%. The experiment was conducted in duplicate (two wells per treatment group), and the average of the data of two wells was used for the analysis.

Relative FGFR3 phosphorylation percentage (%) = (signal amount in the drug solution-added well)/(signal amount in the control group) x 100

The $IC_{50}$ value (50% inhibition concentration) was calculated as the concentration at which 50% of inhibition was achieved relative to the control group.

In 293T cell lines in which the FGFR3 wild-type (WT) or mutants (G380R, N540K, or K650E) were individually expressed, Compound 1 showed the following inhibitory activity (Table 1).

Table 1

| FGFR3 | Inhibition of FGFR3 phosphorylation $IC_{50}$ (nM) |
|---|---|
| WT | 3.2 |
| G380R | 6.1 |
| N540K | 19.8 |
| K650E | 7.4 |

Example 2: Bone extension effect of Compound 1 using achondroplasia model mouse

FGFR3 ACH mice (Naski, M.C. et al., Development, 1998, 125(24): 4977-88; hereinbelow, simply referred to as ACH mice) were used. FVB strain ACH mice were subjected to artificial insemination to produce a large number of F1 hybrid mice. Genomic DNA was then extracted, and the gene type was determined by a PCR method. Compound 1 was dissolved in 0.5% HPMC to prepare solutions with different concentrations. According to the individual body weight at the administration date, each solution was administered at 10 mL/kg so that the dose of Compound 1 was 0.1 mg/kg, 1 mg/kg, or 3 mg/kg. The mice were divided into a Compound 1 administration group and a control group (vehicle = 0.5% HPMC administration) (WT and the vehicle administration group: n=5; Compound 1 administration group: n=6), and intraperitoneal injection was performed once a day for 5 days a week (5 days on, 2 days off) on mice at the age of 21 days to 42 days. Mice at the age of 43 days were euthanized, and the weight measurement and the bone (femur, tibia, and ulna) length measurement by X-ray photographing were performed. The X-ray photographing was performed using a Faxitron X-Ray DX-50 (AcroBio Corporation), and the measurements were performed with Image J. For testing significance, a Dunnett's multiple comparison test was conducted. In Figs. 1 to 5, * indicates $p<0.05$, ** indicates $p<0.01$, and *** indicates $p<0.001$.

As shown in Figs. 1 to 5, at the time of measurement (43 days old), the concentration-dependent bone extension effect was observed in the Compound 1 administration group, as compared to the vehicle administration group.

Subsequently, the femur was isolated from the mice after dosing. The cartilage growth plate was then stained with safranin 0, and the length thereof was measured. The results are shown in Fig. 4. The average width of the growth plate of ACH mice was 148.3. In the group in which Compound 1 was administered to ACH mice, the average width was 206.4. As compared to the vehicle administration group, elongation was confirmed in the Compound 1 administration group.

Example 3: Bone extension effect of Compound 1 using achondroplasia model mice

Using the same method as in Example 2, the dose of Compound 1 (male mice: 1 mg/kg, 3 mg/kg, or 10 mg/kg; female mice: 1 mg/kg, 3 mg/kg, or 6 mg/kg) was changed, and a bone extension effect was evaluated using both male and female mice (n=10). For testing significance, a Dunnett's multiple comparison test was conducted. In Figs. 6 to 11, * indicates $p<0.05$, ** indicates $p<0.01$, *** indicates $p<0.001$, and **** indicates $p<0.0001$.

As shown in Figs. 6 to 11, at the time of measurement (43 days old), the concentration-dependent bone extension effect was observed in the Compound 1 administration group as compared to the vehicle administration group.

Example 4: Evaluation of drug efficacy in disease iPS cell models

iPS cell lines were established from dermal fibroblasts of patients with thanatophoric dysplasia type I (TD1, R248C) and cartilage intangibility (ACH, G380A), and dermal fibroblasts of healthy individuals, and induced their differentiation into chondrocytes. Cartilage tissue was formed from healthy iPS cells, whereas a cartilage component was reduced in tissue induced from TD1-iPS cells and tissue induced from ACH-iPS cells (Yamashita et al., Nature, 2014, 513 (7519): 507-11).

In the differentiation induction system, 1 nM of Compound 1 was added every time the medium was changed (medium was changed once every 2 to 7 days) from the third day after the start of differentiation induction, and an analysis was performed at the $10^{th}$ week of differentiation induction. Rosuvastatin was used as a positive control. As evaluation items, tissue section staining (Safranin staining), and the expression of mRNAs of COL2A1 and Aggrecan (ACAN) were used as indexes. For testing significance, an unpaired test was conducted. In Fig. 13, * indicates $p<0.05$.

As shown in Fig. 12, by the addition of Compound 1, a positive safranin staining image was observed in which glycosaminoglycans, which are constituent components of a cartilage extracellular matrix, were stained.

Further, as shown in Fig. 13, the expression of mRNAs of COL2A1 and ACAN was increased in the Compound 1-added cells, as compared to the cells free of Compound 1.

Sequence Listing:

P20-312WO_PCT_ Treatment of Cartilage Dysplasia_20210127_180232_2.txt

## Claims

1. A pharmaceutical composition for treating cartilage dysplasia, comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof.

2. The pharmaceutical composition according to claim 1, wherein the cartilage dysplasia is achondroplasia, cartilage hypoplasia, or thanatophoric dysplasia.

3. The pharmaceutical composition according to claim 1 or 2, wherein the cartilage dysplasia is achondroplasia.

4. The pharmaceutical composition according to any one of claims 1 to 3, wherein the cartilage dysplasia is cartilage dysplasia having an FGFR3 mutation.

5. The pharmaceutical composition according to claim 4, wherein the FGFR3 mutation is a mutation of the $248^{th}$ arginine, $380^{th}$ glycine, $540^{th}$ asparagine, or $650^{th}$ lysine in the FGFR3.

6. The pharmaceutical composition according to claim 4, wherein the cartilage dysplasia having an FGFR3 mutation is achondroplasia having an FGFR3 mutation.

7. The pharmaceutical composition according to claim 6, wherein the achondroplasia is achondroplasia having an

FGFR3 mutation in which the 380th glycine in the FGFR3 is mutated to arginine.

8. The pharmaceutical composition according to claim 4, wherein the cartilage dysplasia having an FGFR3 mutation is cartilage hypoplasia having an FGFR3 mutation.

9. The pharmaceutical composition according to claim 8, wherein the cartilage hypoplasia is cartilage hypoplasia having an FGFR3 mutation in which the 540th asparagine in the FGFR3 is mutated to lysine.

10. The pharmaceutical composition according to claim 4, wherein the cartilage dysplasia having an FGFR3 mutation is thanatophoric dysplasia having an FGFR3 mutation.

11. The pharmaceutical composition according to claim 10, wherein the thanatophoric dysplasia is thanatophoric dysplasia having an FGFR3 mutation in which the 248th arginine in the FGFR3 is mutated to cysteine, or an FGFR3 mutation in which the 650th lysine in the FGFR3 is mutated to glutamic acid.

12. An agent for cartilage dysplasia treatment comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof.

13. 1-[(3S)-3-[4-Amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof for use in the treatment of cartilage dysplasia.

14. Use of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof for the treatment of cartilage dysplasia.

15. Use of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof in the manufacture of a medicament for the treatment of cartilage dysplasia.

16. A method of treating cartilage dysplasia, comprising the step of administering an effective amount of 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof to a cartilage dysplasia patient.

17. A commercial package comprising 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof as an active ingredient together with instructions for using the 1-[(3S)-3-[4-amino-3-[2-(3,5-dimethoxyphenyl)ethynyl]-1H-pyrazolo[3,4-d]pyrimidin-1-yl]-1-pyrrolidinyl]-2-propen-1-one or a pharmaceutically acceptable salt thereof for the treatment of cartilage dysplasia in a subject.

Fig. 1

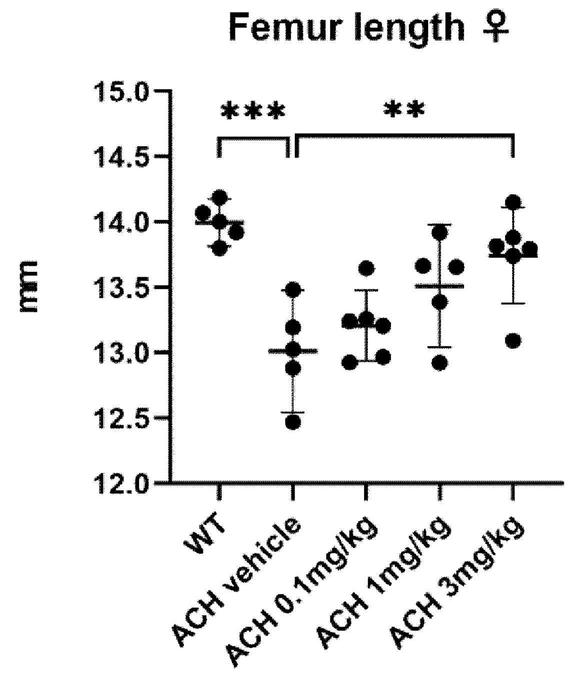
Femur length ♀
15.0
14.5
14.0
13.5
13.0
12.5
12.0
mm
***
**
WT
ACH vehicle
ACH 0.1mg/kg
ACH 1mg/kg
ACH 3mg/kg

Fig. 2

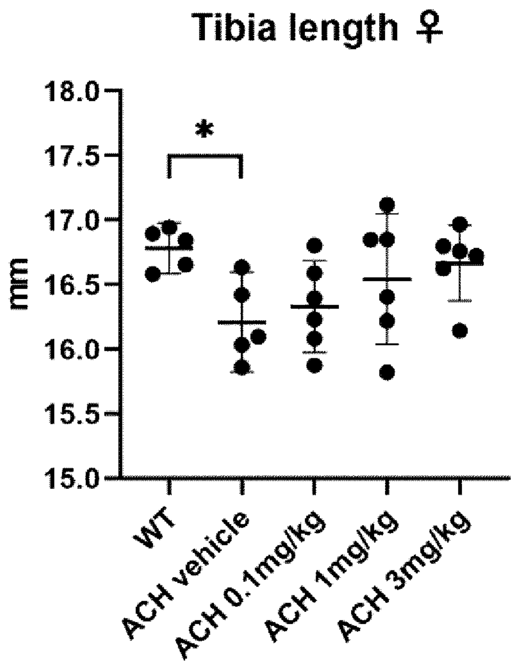
Tibia length ♀
18.0
17.5
17.0
16.5
16.0
15.5
15.0
mm
*
WT
ACH vehicle
ACH 0.1mg/kg
ACH 1mg/kg
ACH 3mg/kg

Fig. 3

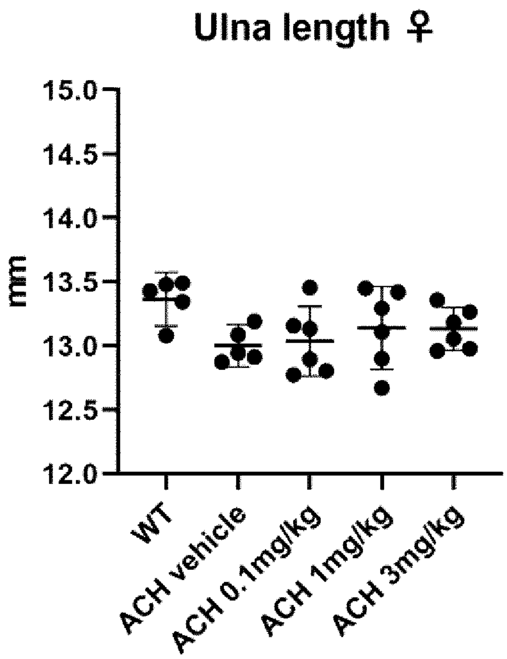
Ulna length ♀
15.0
14.5
14.0
13.5
13.0
12.5
12.0
mm
WT
ACH vehicle
ACH 0.1mg/kg
ACH 1mg/kg
ACH 3mg/kg

Fig. 4

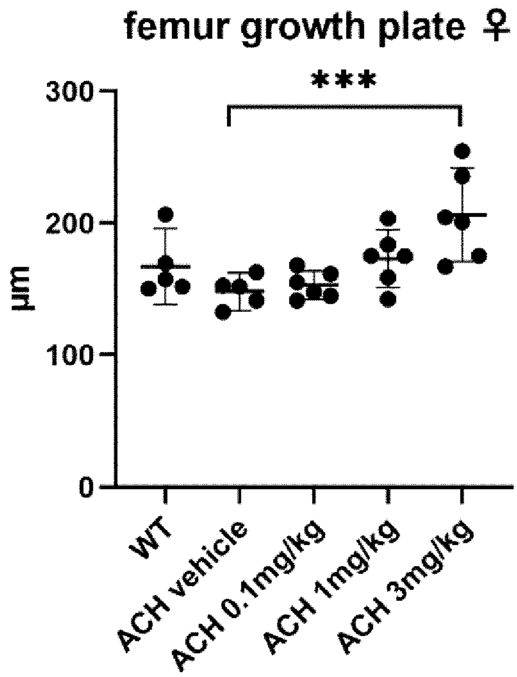
femur growth plate ♀
***
300
200
100
0
µm
WT
ACH vehicle
ACH 0.1mg/kg
ACH 1mg/kg
ACH 3mg/kg

Fig. 5

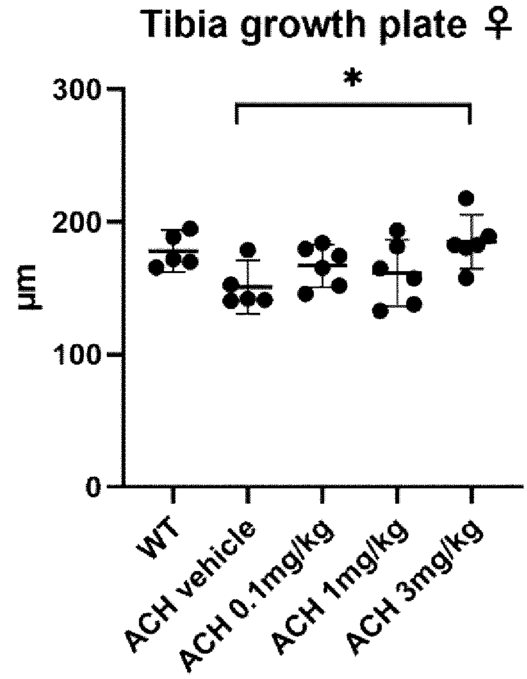
Tibia growth plate ♀
*
300
200
100
0
μm
WT
ACH vehicle
ACH 0.1mg/kg
ACH 1mg/kg
ACH 3mg/kg

Fig. 6

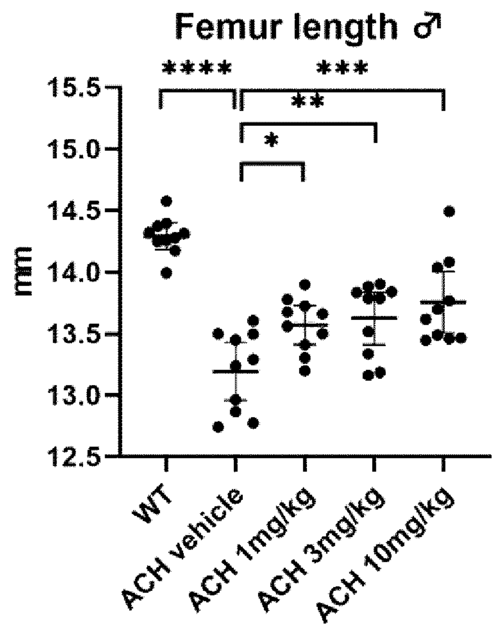
Femur length ♂
****
***
**
*
15.5
15.0
14.5
14.0
13.5
13.0
12.5
mm
WT
ACH vehicle
ACH 1mg/kg
ACH 3mg/kg
ACH 10mg/kg

Fig. 7

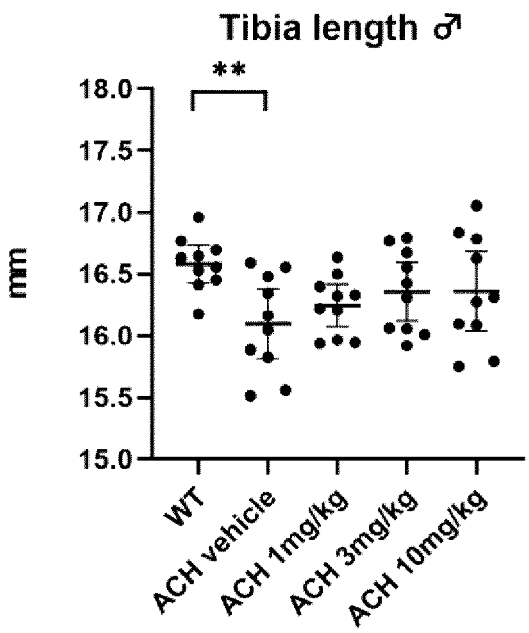
Tibia length ♂
**
18.0
17.5
17.0
16.5
16.0
15.5
15.0
mm
WT
ACH vehicle
ACH 1mg/kg
ACH 3mg/kg
ACH 10mg/kg

Fig. 8

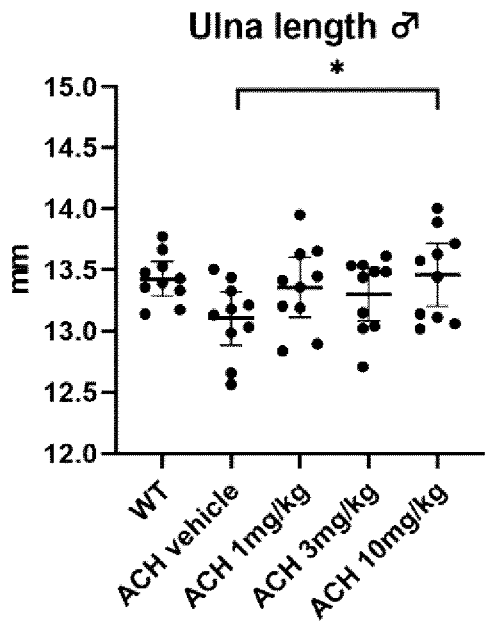
Ulna length ♂
*
15.0
14.5
14.0
13.5
13.0
12.5
12.0
mm
WT
ACH vehicle
ACH 1mg/kg
ACH 3mg/kg
ACH 10mg/kg

Fig. 9

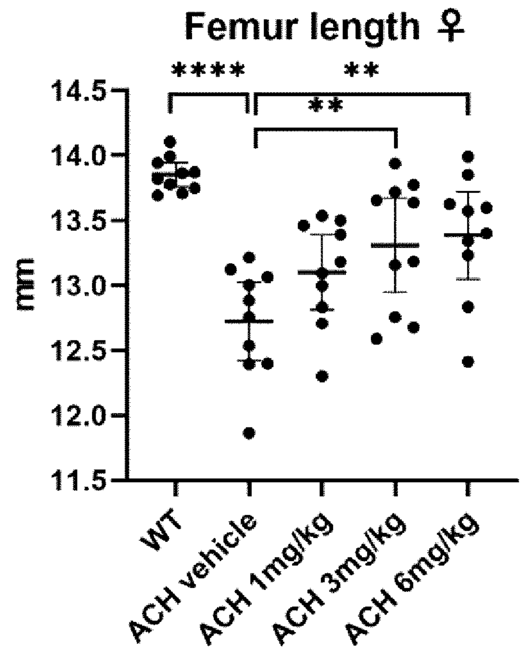
Femur length ♀
****
**
**
14.5
14.0
13.5
13.0
12.5
12.0
11.5
mm
WT
ACH vehicle
ACH 1mg/kg
ACH 3mg/kg
ACH 6mg/kg

Fig. 10

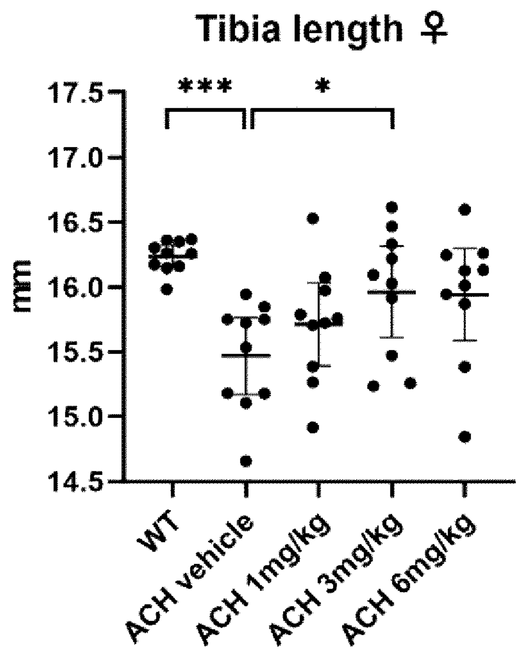
Tibia length ♀
***
*
17.5
17.0
16.5
16.0
15.5
15.0
14.5
mm
WT
ACH vehicle
ACH 1mg/kg
ACH 3mg/kg
ACH 6mg/kg

Fig. 11

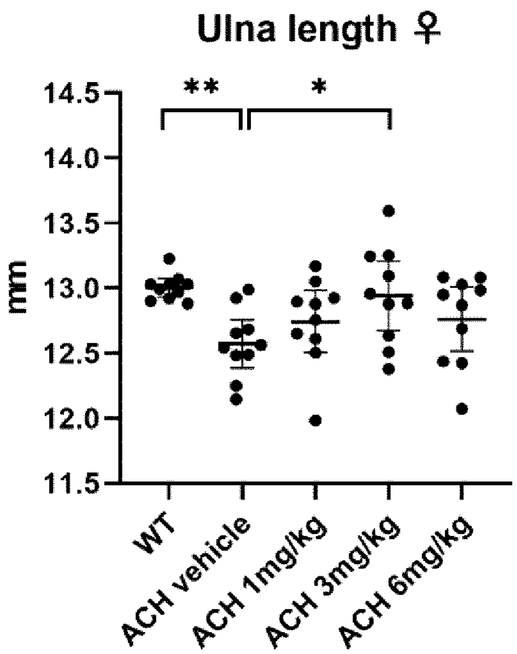
Ulna length ♀
**
*
14.5
14.0
13.5
13.0
12.5
12.0
11.5
mm
WT
ACH vehicle
ACH 1mg/kg
ACH 3mg/kg
ACH 6mg/kg

Fig. 12

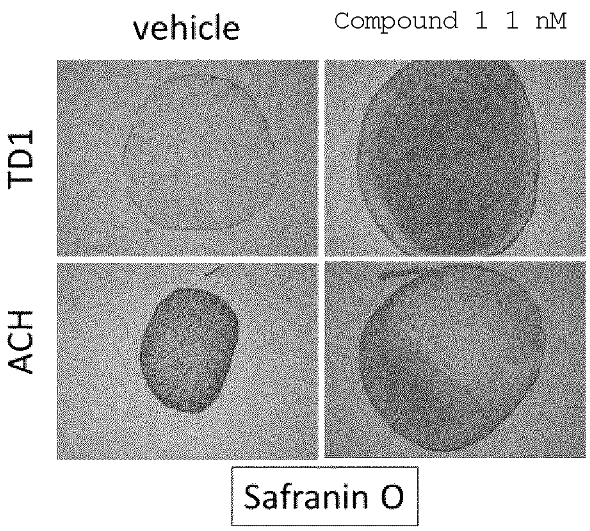
vehicle
Compound 1 1 nM
TD1
ACH
Safranin O

Fig. 13

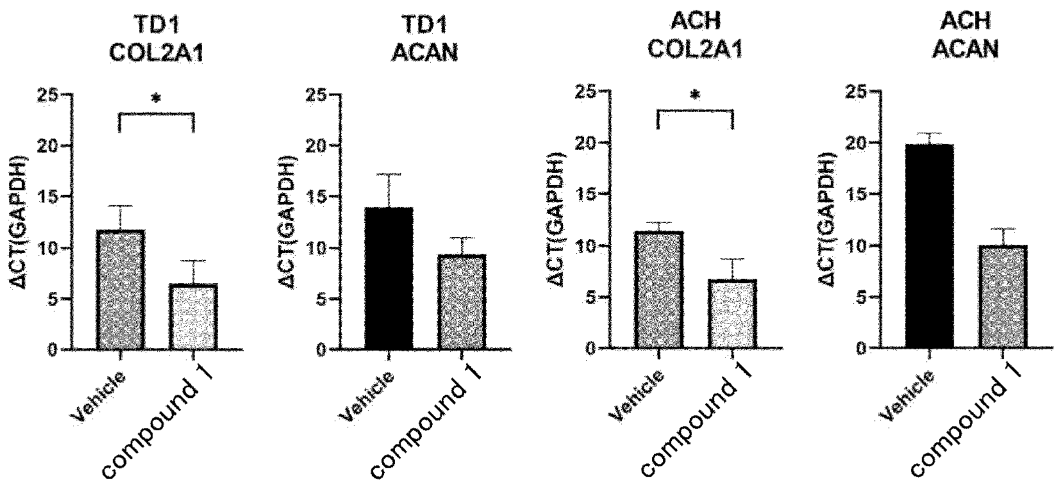
TD1
COL2A1
ΔCT(GAPDH)
25
20
15
10
5
0
*
Vehicle
compound 1
TD1
ACAN
ΔCT(GAPDH)
Vehicle
compound 1
ACH
COL2A1
ΔCT(GAPDH)
*
Vehicle
compound 1
ACH
ACAN
ΔCT(GAPDH)
Vehicle
compound 1

## INTERNATIONAL SEARCH REPORT

International application No.
PCT/JP2021/003133

A. CLASSIFICATION OF SUBJECT MATTER
A61K 31/519(2006.01)i; A61P 19/08(2006.01)i; C07K 14/715(2006.01)i
FI: A61K31/519; A61P19/08; C07K14/715 ZNA
According to International Patent Classification (IPC) or to both national classification and IPC

B. FIELDS SEARCHED
Minimum documentation searched (classification system followed by classification symbols)
A61K31/519; A61P19/08; C07K14/715

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2015/008844 A1 (TAIHO PHARMACEUTICAL CO., LTD.) 22 January 2015 (2015-01-22) test example 4, table 23, example compound 2 | 13, 17<br>1-17 |
| Y | GARCIA, S. et al., "Postnatal soluble FGFR3 therapy rescues achondroplasia symptoms and restores bone growth in mice", Sci Transl Med, 2013, vol. 5, no. 203, Article 203ra124 abstract, introduction | 1-17 |
| Y | KOMLA-EBRI, D. et al., "Tyrosine kinase inhibitor NVP-BGJ398 functionally improves FGFR3-related dwarfism in mouse model", J Cl in Invest, 2016, vol. 126, no. 5, pp. 1871-84 abstract | 1-17 |
| Y | JP 2016-501520 A (ROCHE INNOVATION CENTER COPENHAGEN A/S) 21 January 2016 (2016-01-21) claims 1-15, fig. 1-7, example 7 | 1-17 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 March 2021 (08.03.2021) | 16 March 2021 (16.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2021/003133

C (Continuation). DOCUMENTS CONSIDERED TO BE RELEVANT

| Category* | Citation of document, with indication, where appropriate, of the relevant passages | Relevant to claim No. |
|---|---|---|
| Y | RAMASWAMI, U, et al., "Genotype and phenotype in hypochondroplasia", J Pediatr, 1998, vol. 133, no. 1, p. 99-102 abstract | 1-5, 8, 9, 12-17 |
| Y | KIMURA, T. et al., "The incidence of thanatophoric dysplasia mutations in FGFR3 gene is higher in low-grade or superficial bladder carcinomas", Cancer, 2001, vol. 92, no.10, pp. 2555-61 abstract | 1-5, 10-17 |

Form PCT/ISA/210 (continuation of second sheet) (January 2015)

**INTERNATIONAL SEARCH REPORT**
Information on patent family members

International application No.
PCT/JP2021/003133

| Patent Documents referred in the Report | Publication Date | Patent Family | Publication Date |
|---|---|---|---|
| WO 2015/008844 A1 | 22 Jan. 2015 | US 2016/0136168 A1<br>test example 4, table 23, example compound 2<br>US 2019/0015417 A1<br>EP 3023101 A1 | |
| JP 2016-501520 A | 21 Jan. 2016 | US 2015/0307886 A1<br>claims 1-15, fig. 1-7, example 7<br>WO 2014/080004 A1<br>EP 2922955 A1<br>CA 2889993 A1<br>KR 10-2015-0088305 A<br>CN 104903448 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020014260 A
- WO 2013108809 A
- WO 2015008844 A
- WO 2015008839 A


### Non-patent literature cited in the description


- *Dev. Dyn.,* April 2017, vol. 246 (4), 291-309
- *Am. J. Hum. Genet.,* December 2000, vol. 67 (6), 1411-21
- *Nature,* 15 September 1994, vol. 371 (6494), 252-4
- *Nat. Genet.,* July 1995, vol. 10 (3), 357-9
- *Am. J. Med. Genet.,* 03 May 1996, vol. 63 (1), 148-54
- *J. Clin. Invest.,* 2016, vol. 126 (5), 1871-1884
- **NASKI, M.C. et al.** *Development,* 1998, vol. 125 (24), 4977-88
- **YAMASHITA et al.** *Nature,* 2014, vol. 513 (7519), 507-11